# EUROPEAN PATENT SPECIFICATION

(11) **EP 0 815 867 B1**
(45) Date of publication and mention of the grant of the patent: **12.11.2003**
(21) Application number: 97900753.1
(22) Date of filing: 22.01.1997
(51) Int. Cl.: A61K 35/50, C12N 5/06, A61P 25/28

(54) **CELLS FOR TREATING DEMENTIA**
ZELLEN ZUR BEHANDLUNG VON DEMENZ
CELLULES DESTINEES AU TRAITEMENT DE LA DEMENCE

(30) Priority: 23.01.1996 JP 2848396; 01.03.1996 JP 7110096
(43) Date of publication of application: 07.01.1998
(73) Proprietor: SRL, INC., Tachikawa-shi, Tokyo 190 (JP)
(72) Inventor: SAKURAGAWA, Norio, Kodaira-shi, Tokyo 187 (JP)
(74) Representative: Kiddle, Simon John
(86) International application number: JP9700132
(87) International publication number: WO97026902

(56) References cited:
- EP-A- 0 333 328
- WO-A-81/02674
- WO-A-92/06702
- FISHER LJ ET AL.: "In vivo production and release of acetylcholine from primary fibroblasts genetically modified to express choline acetyltransferase" J NEUROCHEMISTRY, vol. 61, no. 4, 1993, pages 1323-1332, XP000983400 New York
- FISHER LJ ET AL.: "Cells engineered to produce Acetylcholine: Therapeutic potential for Alzheimer's Disease" ANN N Y ACAD SCI, vol. 695, 1993, pages 278-284, XP000983856 New York
- SALVATERRAPM: "Molecular Biology and Neurobiology of Choline Acetyltransferase" MOLECULAR NEUROBIOLOGY, vol. 1, no. 3, 1987, pages 247--280, XP000983533
- SAKURAGAWA N ET AL.: "Expression of markers for both neuronal and glial cells in human amniotic epithelial cells" NEUROSCIENCE LETTERS, vol. 209, no. 1, May 1996 (1996-05), pages 9-12, XP002159826
- ARCH. NEUROL., 1993, Vol. 50, SUHR S.T. et al., "Gene Therapy for Neurologic Disease", pages 1252-1268, XP002944353.
- J. NEUROCHEM., 1993, Vol. 61, No. 4, FISHER L.J. et al., "In Vivo Production and Release of Acetylcholine from Primary Fibroblasts Genetically Modified to Express Choline Acetyltransferase", pages 1323-1332, XP000983400.
- EXP. NEUROL., 1993, Vol. 123, No. 2, SHENG J.G. et al., "Dopaminergic Neuronal Sprouting and Behavioral Recovery in Hemi-Parkinsonian Rats After Implantation of Amnion Cells", pages 192-203, XP002944354.
- CHEMICAL ABSTRACTS, Vol. 73, No. 42842p, (1970), XP002944395 & Z. Klin. Chem. Klin. Biochem., vol. 8, no. 2, 1970,pages 145 - 148.

## Description

### TECHNICAL FIELD

The present invention relates to cells for treating dementia.

### BACKGROUND ART

It has been proposed to transplant neural progenitor cells to the central nervous system to directly supply therapeutic gene products, thereby curing nervous function disorders (Snyder, E.Y. et al., Cell 68, 33-51 (1992); Groves, A.K. et al., Nature 362, 453-455 (1993); Renfranz, P.J. et al., Cell 66, 713-729 (1991)). Recently, Snyder et al. reported that accumulation of lysosomes in the brain of MPS VII mouse is cured by transplanting neural progenitor cells.

On the other hand, Alzheimer's disease is a dementia exhibiting neurofibriallary tangle (NFT); the domains known as senile plaques in which neurites are degenerated; the single granule having a high electron density in the nerve cell, which is called granulovacuolar degeneration (GVD); and acidophilic inclusion bodies called Hirano endoplasmic reticulum (C.U. M. Smith, "Smith Molecular Neurobiology", Ohm, p.419). Another important symptom of Alzheimer's disease is the large decrease in choline acetyltransferase in especially hippocampus.

Choline acetlytransferase is normally expressed in neurones that use acetylcholine as a neurotransmitter, and these cholinergic neurones are damaged in Alzheimer's disease. Salvaterra discussed the 'cholinergic hypothesis' of a causal relationship in Alzheimer's disease between the decrease in choline acetyltransferase and the loss of cholinergic neurones proposed to have a central role in memory (Salvaterra, P. M., *Molecular Neurobiology* **1** 247-280 (1987)). It is known that blocked neural transmission is recovered by administering acetylcholine to the damaged portion (e.g., senile plaque in the hippocampus) (Suhr ST, Gage FH, Gene therapy for neurologic disease, Arch Neurol 1993; 50:1252-1268). it is also known that by transplanting primary fibroblast cells having Drosophila choline acetyltransferase gene to hippocampus of normal rats, acetylcholine is produced by these cells (Fisher LJ et al., In vivo porduction and release of acetylcholine from primary fibroblasts genetically modified to express choline acetyltransferase, J. Neurochem. 1993; 61:1323-1332).

Intracerebral implantation of a patient's own cells genetically modified to express choline acetyltransferase has been advocated as a means of ameliorating the cognitive impairment associated with Alzheimer's disease. However, without the use of neurones or neural progenitor cells, this approach only delivers the depleted chemical, and cannot provide cells that are functionally incorporated into the brain to replace the damaged neurones. (Fisher, L. J., *Annals New York Academy of Sciences* 278-284 (1993).)

WO 92/06702 disclosed methods of grafting cells into the brain of a mammalian subject, suitable for treating neurodegenerative diseases such as Alzheimer's disease. The method involved attaching the cells to a support matrix and implanting this matrix into the brain. Various cell types were used in the method, including cells grown *in vitro.*

EP 0 333 328 A2 disclosed the use of amniotic epithelial cells for tissue repair *in vivo,* such as the treatment of cartilage-denuded joints. Human amniotic cells were transplanted into the rabbit knee joint, rabbit Achilles tendon, rat liver and rat abdominal cavity without inducing a major immune response. The amniotic cells became incorporated into the host tissue. Thus, it was shown that amniotic cells at least partially retain the pluripotent characteristic of being able to differentiate into other tissues.

### DISCLOSURE OF THE INVENTION

Therefore, it is thought that by transplanting cells capable of producing choline acetyltransferase, which can take without being rejected by the body, to the brain of a dementia patient, choline acetyltransferase and, in turn, acetylcholine is supplied, so that the dementia is cured or at least lightened. In case of transplanting cells to the brain, to prevent rejection reaction, the cells to be transplanted are preferably of human origin. However, no human neural progenitor cells have been reported except for fetal brain cells. Fetal brain cells, however, cannot be secured stably.

An object of the present invention is to provide cells for treating dementia, which can be secured stably, which are capable of producing choline acetyltransferase when transplanted to the brain, and which can take in the brain without being subjected to rejection reaction by the body.

The present inventors intensively studied to discover that human amniotic epithelial cells are neural progenitor cells and produce choline acetyltransferase when transplanted to the brain and that antigenicity thereof is small so that they can take in the brain, thereby completing the present invention. Thus, the present invention provides use of human amniotic cells as cells for treating dementia. This invention comprises the use of human amniotic epithelial cells capable of producing choline acetyltransferase in the preparation of a medicament for treating dementia, e.g. Alzheimer's disease. The human amniotic epithelial cells may be cultured. Preferably, the medicament of the invention is administered to the patient's brain by transplantation, ideally to the hippocampus, basal ganglia or the brain striate body.

By transplanting the cells for treating dementia according to the present invention to the brain, choline acetyltransferase and, in turn, acetylcholine is produced in the brain, so that dementia such as Alzheimer's disease is lightened. Since human amniotic epithelial cells used in the present invention may be obtained from human placentae, the cells may be stably secured without ethical problems. Further, since human amniotic epithelial cells do not express Class II surface antigen and expression of class I surface antigen is small, they can take in the brain without being subjected to rejection reaction.

### BRIEF DESCRIPTION OF THE DRAWINGS

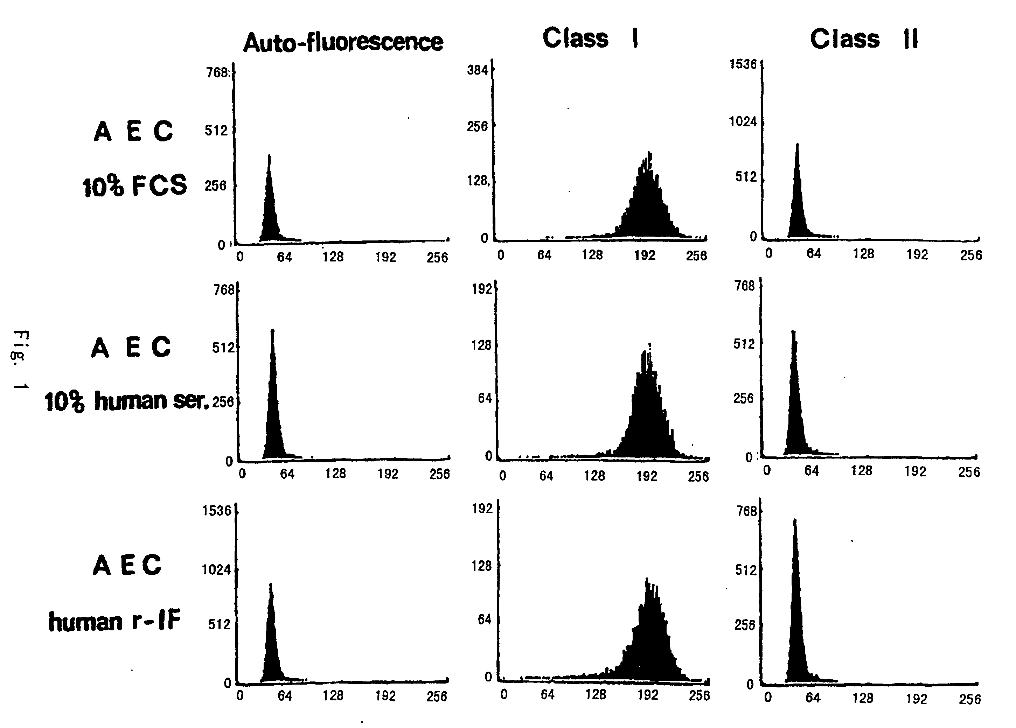

Fig. 1 shows the results of flow cytometry of the cells according to the present invention or the cells according to the present invention treated with γ-interferon, which were stained with an anti-class I antibody or an anti-class II antibody.

### BEST MODE FOR CARRYING OUT THE INVENTION

Human amniotic epithelial (hereinafter also referred to as "HAE") cells are formed from amnioblasts, separated from the epiblast at about the 8th day after fertilization. To check whether HAE cells are multipotential neural progenitor cells, immunocytochemical studies were made to detect some specific markers. HAE cells display positive immunoreactivity to neurofilament proteins, microtubule-associated protein 2, microtubule-associated protein 2 kinase, A2B5, vimentin and glial fibrillary acidic protein. Among the oligodendrocyte markers, the HAE cells demonstrate immunoreactivity to 2':3'-cyclic nucleotide 3'-phosphodiesterase, galactocerebroside and myelin basic protein. From these data, it is thought that HAE cells express antigens of neurons, type 2 astrocytes and oligodendrocytes. The experimental methods for checking immunoreactivities to the above-mentioned markers and the results thereof are described in Reference Example 1 below.

The present inventor discovered that human amniotic cells do not express class II surface antigen, and expression of class I surface antigen thereof is also small (see Reference Example 2 below, Sakuragawa et al., Cell Transplantation, Vol.4, No.3, pp.343-346, 1995). Therefore, human amniotic cells are not substantially subjected to rejection reaction. As is apparent from Reference Example 1 below, the present inventor discovered that HAE cells have markers of nervous system cells. Further, as will be concretely described in Example 1 below, HAE cells produce choline acetyltransferase when transplanted to the brain. Therefore, by transplanting HAE cells to the brain, the choline acetyltransferase produced by the HAE cells is supplied to the brain cells, so that dementia is improved and rejection reaction by the transplantation does not occur. Therefore, HAE cells are useful as cells for treating dementia. Further, since HAE cells can be obtained from placentae, they can stably be obtained.

The cells for treating dementia according to the present invention comprises HAE cells. They may contain HAE cells alone or may contain other cells and/or other pharmaceutically acceptable components, which do not adversely affect the present invention, in addition to HAE cells. When using the cells for treating dementia according to the present invention, HAE cells are transplanted to the brain. Although the number of the HAE cells to be transplanted may appropriately be selected depending on the symptom, usually, 10⁴ to 10⁶ cells are transplanted. Although the site of the brain to which the cells are transplanted is not restricted, the site is preferably the portions damaged by the dementia. For example, in case of Alzheimer's disease, hippocampus, basal ganglia and brain striate body are preferred.

The present invention will now be described in more concretely by way of examples. It should be noted that the examples are presented for the illustration purpose only and should not be interpreted in any restrictive way.

### Reference Example 1 Detection of Nerve Cell Markers on HAE Cells

To check whether HAE cells are multipotential neural progenitor cells, immunocytochemical methods for detecting various development specific markers were employed. As the nerve cell markers, neurofilament proteins (NF), microtubule-associated protein 2 (MAP2) and microtubule-associated protein 2 kinase (MK) were employed. To identify neuroglia cells, antibodies to glial fibrillary acidic protein (GFAP), myelin basic protein (MBP), galactocerebroside (GC) and 2':3'-cyclic nucleotide 3'-phosphodiesterase (CNPase) were used. Vimentin was used as a marker for undeveloped neuroglia, neuron progenitor cells, O-2A progenitor cells and type 2 astrocytes. A2B5 monoclonal antibody was used for detecting A2B5(+) neurons, O-2A progenitor cells and type 2 astrocytes.

### Materials and Methods

(1) The HAE cells were prepared from a placenta obtained from an uncomplicated elective caesarean section and then cultured in RPMI-1640 medium supplemented with 10% fetal calf serum under a humidified atmosphere of 5% CO₂ in air at 37°C. Cells grown on coverslips seated in a twenty-four well dish were immunocytochemically stained as follows. The cells were fixed with 4.0% paraformaldehyde in PBS(-) for 15 minutes at room temperature. Then the cells were incubated with primary antibodies for 1 hour after blocking with 5% normal goat serum and with 3% BSA. The primary antibodies used were mouse monoclonal antibodies to NF (1:20, Serotec), MAP2 (1:20, Chemicon), MK (1:500, Pharmingen) and to GFAP (1:5, Serotec) and mouse IgG (3 µg/ml, Vector). Then the coverslips were washed with PBS(-) and treated with a secondary antibody, biotinylated anti-mouse IgG antibody (1:200, Vector) for 1 hour, and then with peroxidase-conjugated streptavidin (Nichirei) for 1 hour. The coverslips were washed again, followed by application of 0.5 mg/ml diaminobenzidine (Dojin) in 0.05 M Tris-HCl, pH 7.2, to which hydrogen peroxide (0.006%) was added immediately before application to the slides. The slides were counterstained with Harris hematoxylin for 30 seconds.
(2) Immunocytochemical staining was performed in the same manner as in (1). The primary antibodies used were mouse monoclonal antibodies to vimentin (1:50, DAKO) and to CNPase (1:50, Sigma). The supernatant of the culture medium containing the hybridomas secreting mouse monoclonal antibody A2B5 was used at a dilution of 1:5.
(3) Immunocytochemical study was made using the indirect fluorescent antibody technique. Rabbit polyclonal antibody to galactocerebroside (1:50, SIGMA) and rabbit polyclonal antibody to MBP (1:200, DAKO) were used.
(3) Immunoblotting was done in accordance with a known method (Mizuguchi et al., Brain Res. 649, 197-202(1994)). That is, Immobilon polyvinylidene difluoride membrane (Millipore) was incubated with primary antibodies. The primary antibodies used were antibodies to MAP2 (1:30), MK (1:500) and to GFAP (1:500) as in (1). Then the membrane was treated with biotinylated goat anti-rabbit IgG (Vector, 1:500) and peroxidase-conjugated streptavidin (Zymed, 1:500). The immunoproduct was visualized by treatment with a solution containing 0.03% 3,3'-diaminobenzidine and 0.006% hydrogen peroxide in 50 mM Tris-HCl, pH 7.4.

### Results

HAE cells displayed strong immunoreactivity to NF, MAP2 and MK. Antigenicity to MAP2 and MK was also detected by immunoblotting, which indicated the presence of bands of 300 kD and 42 kD, respectively. Immunoreactivity to vimentin was found in the cells. Vimentin is initially expressed in nearly all neuronal precursor cells *in vivo,* and is gradually replaced by neurofilament subunits shortly before the immature neurons become postmitotic. Microtubules arise early in neuronal development, usually long before neurofilaments. HAE cells were shown to contain NF, MAP2 and vimentin, suggesting that these cells differentiated to acquire the expression of neuronal markers.

GFAP was positively stained in the HAE cells and a single immunoreactive band was detected on the blots of the HAE cell extracts, indicating presence of GFAP as in the types 1 and 2 astrocytes. Also HAE cells displayed strong immunoreactivity to the surface marker A2B5. A2B5-positive cells are neurons and O-2A lineage cells such as type 2 astrocytes and immature oligodendrocytes. HAE cells exhibited immunoreactivities to both A2B5 and GFAP like the type 2 astrocytes.

With regard to the markers for developing oligodendroglia cells, CNPase, Gal C and MBP were positively stained. Further, HAE cells expressed vimentin which is an intermediate filament protein. Judging from the flow chart on the differentiation of the O-2A lineage (Dubois-Daloq, M.EMBO J. 6:2587-2595, (1987)), HAE cells exhibiting positive reactivities to the antibodies to A2B5, vimentin, CNPase, GFAP, Gal C and MBP possess the properties of both oligodendrocytes and type 2 astrocytes.

As described above, it was proved that HAE cells expressed phenotypes of both neuronal and neuroglial cells.

### Reference Example 2

Amniotic epithelial layer was prepared by a known method (Akle et al., Lancet II: 1003-1005, 1981) from a placenta obtained from caesarean section. The separated amniotic sheet was dissected into several pieces and then treated with trypsin-EDTA (trypsin concentration: 0.125%) for 15 minutes. The isolated cells were collected by centrifugation, and then cultured in RPMI-1640 medium supplemented with 10% fetal calf serum under an atmosphere of 5% CO₂ in air at 37°C. Eighty cm² tissue culture flasks were used and near confluent cells were used for experiments.

The following mouse monoclonal antibodies were used: anti-human HLA class I antigen monoclonal antibody (DAKO A/S, Denmark, clone W6/32) and anti-human HLA-DR class II antigen, α-chain monoclonal antibody (DAKO, clone TAL.1B5). Also horse radish peroxidase (HRPO)-labeled goat F(ab')₂ anti-mouse Ig's (G+L) antibody (TAGO, INC. CA, USA) was used for enzyme-labeled antibody method. For flow cytometry, monoclonal anti-human HLA class I antigen (as mentioned above) and monoclonal anti-human HLA-DR (DAKO, clone CR3/43) were used for detection of class I and II antigens as well as fluorescein conjugated anti-mouse immunoglobulin F(ab)₂ fraction (Silenus Lab. Hawthorn Victoria, Australia).

For flow cytometry, near confluent human amniotic cells from 80 cm² tissue culture flasks were harvested by treatment with trypsin-EDTA and then washed with cold PBS. The cells were subsequently incubated at 4°C with the primary antibodies for 30 minutes and then resuspended with the FITC-conjugated second antibodies for 30 minutes at 4°C. The cells were then analyzed immediately with a flow cytometer (FCM-1D-JASCO Co., Tokyo).

To rule out effects of γ-interferon on the induction of class I and class II antigens, the human amniotic cells prepared as described above were treated with γ-interferon (100 U/ml medium with 10% human serum of blood type AB, Sigma, USA) at 37°C for 3 days. The resulting cells were analyzed by flow cytometry as described above.

The results of the flow cytometry are shown in Fig. 1. In Fig. 1, the abscissa indicates fluorescence intensity and the ordinate indicates the number of cells. Fig. 1B shows the results of the cells cultured in 10% human serum for 3 days. As shown in Fig. 1A, the cultured amniotic cells did not express the class II antigen at all on their surfaces as judged on comparison with the histogram for these cells stained with nonspecific mouse IgG1. Judging from its lower fluorescence intensity than that of lymphocytes as controls (not shown), the class I antigen was expressed slightly. Also, it was proved that the γ-interferon treatment neither increased the class I expression nor induced class II antigens (Fig. 1C).

On the other hand, a preparation of amniotic epithelial cells was incubated with anti-class I primary antibody or anti-class II primary antibody at 37°C for 1 hour. The cells were then incubated with a HRPO-conjugated antibody at 37°C for 1 hour, followed by reacting the resultant with diaminobenzidine for 5 minutes at room temperature to generate color. As a result, the cells were not stained at all when the anti-class II monoclonal antibody was used and slightly stained when the anti-class I monoclonal antibody was used.

### Example 1 Expression of Choline Acetyltranferase in HAE Cells and Production of Choline Acetyltransferase in HAE Cells Transplanted to Rat Brain

Amniotic cells were separated from human placenta and cultured by the method described above (Reference Example 1). Cultured cells on coverslips were immunocytochemically stained with a commercially available anti-choline acetyltransferase antibody (Boehringer Mannheim, 1:5 dilution) by the method as in Reference Example 1. As a result, the cells were stained. The cultured cells were treated with trypsin to prepare a cell suspension (about 5 x 10⁵/5 µl, PBS). Wistar rats (200 - 300 g) were anaesthetized with nembutal and 5 µl of the cell suspension was transplanted to rat brain striate body stereoencephalotomically. Two weeks after the transplantation, the rats were anaesthetized and perfused with 4% paraformaldehyde to attain fixation. Brains were removed and ultra-thin sections were prepared. Using this specimen, immunocytochemical staining was performed with a commercially available anti-choline acetyltransferase antibody (Boehringer Mannheim, 1:5 dilution) as in Reference Example 1. As a result, the cells were stained. From these results, it was proved that HAE cells take in the brain and choline acetyltransferase is produced by the cells which took in the brain.

## Claims

1. Use of human amniotic epithelial cells capable of producing choline acetyltransferase in the preparation of a medicament for treating dementia.

2. Use according to claim 1 wherein said dementia is Alzheimer's disease.

3. Use according to claim 1 or claim 2 wherein the human amniotic epithelial cells are cultured.

4. Use according to any of claims 1 to 3 wherein the medicament is administered to the brain by transplantation.

5. Use according to claim 4 wherein the medicament is transplanted to the hippocampus, basal ganglia or the brain striate body.

## Patentansprüche

1. Verwendung menschlicher amniotischer Epithelzellen, die zur Produktion von Cholin-Acetyltransferase fähig sind, bei der Herstellung eines Medikaments zur Behandlung von Demenz.

2. Verwendung nach Anspruch 1, worin die Demenz die Alzheimer-Krankheit ist.

3. Verwendung nach Anspruch 1 oder 2, worin die menschlichen amniotischen Epithelzellen kultiviert werden.

4. Verwendung nach einem der Ansprüche 1 bis 3, worin das Medikament dem Gehirn durch Transplantation verabreicht wird.

5. Verwendung nach Anspruch 4, worin das Medikament in den Hyppocampus, die Basalganglien oder den Corpus striatum des Gehirns transplantiert wird.

## Revendications

1. Utilisation de cellules épithéliales amniotiques capables de produire de la choline acétyltransférase dans la préparation d'un médicament pour traiter la démence.

2. Utilisation selon la revendication 1 où ladite démence est la maladie de Alzheimer.

3. Utilisation selon la revendication 1 ou la revendication 2 où les cellules épithéliales amniotiques humaines sont cultivées.

4. Utilisation selon l'une quelconque des revendications 1 à 3 où le médicament est administré au cerveau par transplantation.

5. Utilisation selon la revendication 4 où le médicament est transplanté à l'hippocampe, aux ganglions basaux ou au corps strié du cerveau.
